# EUROPEAN PATENT APPLICATION

(11) **EP 0 666 313 A2**
(43) Date of publication of application: **09.08.1995**
(21) Application number: 95300539.4
(22) Date of filing: 27.01.1995
(51) Int. Cl.: C12N 15/54, C12N 9/12, A61K 48/00, C12Q 1/68

(54) **Targeting of telomerase in cancer gene therapy**

(30) Priority: 27.01.1994 US 189151
(71) Applicant: IOWA STATE UNIVERSITY RESEARCH FOUNDATION, INC., Ames, Iowa 50011-3021 (US)
(72) Inventor: Henderson, Eric, Ames, Iowa 50010 (US)
(74) Representative: Bassett, Richard Simon

(57) **Abstract**

The present invention provides for the identification and cloning of the RNA component of the telomerase enzyme, human telomerase RNA (telRNA). In another aspect of invention, there is disclosed a novel tumor cell-specific gene therapy, using the telRNA of the invention for the specific destruction of various types of cancers. The human telomerase RNA (telRNA) gene can be identified (isolated) by direct hybridization using an oligonucleotide probe to screen cultured human tumor cells enriched in telRNA (HeLa cells) and/or by PCR/hybrid selection. Once isolated, the gene encoding the telRNA gene is mutated *in vitro* and cloned into a suitable expression vector. The expression vector can be transfected into human cancer cells, *in vitro* or *in vivo*. In either case, it is expected that the mutant telRNA will compete with the endogenous telRNA in the formation of active telomerase, resulting in the addition of aberrant telomere sequences to the ends of chromosomes. This is predicted to cause genetic instability and rapid cell death.

SEQUENCE LISTING

(1) GENERAL INFORMATION:

(i) APPLICANT: IOWA STATE UNIVERSITY RESEARCH FOUNDATION, INC

(ii) TITLE OF INVENTION: Targeting of Telomerase in
Cancer Gene Therapy

(iii) NUMBER OF SEQUENCES: 15

(iv) CORRESPONDENCE ADDRESS:
(A) ADDRESSEE: ERIC POTTER CLARKSON
(B) STREET: ST MARY'S COURT, ST MARY'S GATE
(C) CITY: NOTTINGHAM
(D) STATE: ENGLAND
(E) COUNTRY: UNITED KINGDOM
(F) ZIP: NG1 1LE

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME:
(B) REGISTRATION NUMBER:
(C) REFERENCE/DOCKET NUMBER:

(ix) TELECOMMUNICATION INFORMATION:
(A) TELEPHONE:
(B) TELEFAX:

(2) INFORMATION FOR SEQ ID NO:1:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:1:

TTGGGGTTG 9

(2) INFORMATION FOR SEQ ID NO:2:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:2:

TTAGGGTTA 9

(2) INFORMATION FOR SEQ ID NO:3:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:3:

TTAGGGTTAG GG 12

(2) INFORMATION FOR SEQ ID NO:4:

(i) SEQUENCE CHARACTERISTICS:
(A) LENGTH: 7 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:4:

CAACAAA 7

(2) INFORMATION FOR SEQ ID NO:5:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:5:

TAACCCTAA 9

(2) INFORMATION FOR SEQ ID NO:6:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:6:

CAACCCCAA 9

(2) INFORMATION FOR SEQ ID NO:7:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:7:

TTGGGGTTG 9

(2) INFORMATION FOR SEQ ID NO:8:

(i) SEQUENCE CHARACTERISTICS:
(A) LENGTH: 6 base pairs

(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:8:

TAATAA 6

(2) INFORMATION FOR SEQ ID NO:9:

(i) SEQUENCE CHARACTERISTICS:
(A) LENGTH: 8 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:9:

TTACCTTA 8

(2) INFORMATION FOR SEQ ID NO:10:

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:10:

TTACCCCTTA 10

(2) INFORMATION FOR SEQ ID NO:11:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:11:

CAACCCCAA 9

(2) INFORMATION FOR SEQ ID NO:12:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:12:

CGACCCCAA 9

(2) INFORMATION FOR SEQ ID NO:13:

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:13:

CAACCCCCAA 10

(2) INFORMATION FOR SEQ ID NO:14:

(i) SEQUENCE CHARACTERISTICS:
(A) LENGTH: 9 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:14:

CAACCTCAA 9

(2) INFORMATION FOR SEQ ID NO:15:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO:15:

CAAAACCCCA AA 12

## Description

### FIELD OF THE INVENTION

The present invention relates generally to cancer gene therapy.

### BACKGROUND OF THE INVENTION

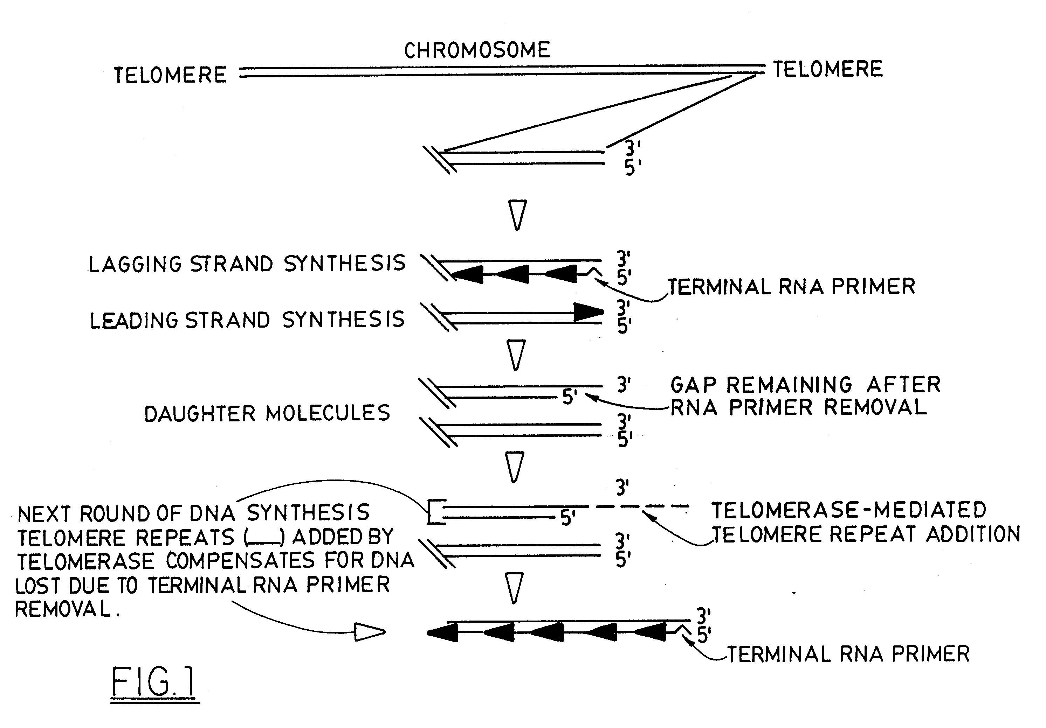

Telomeres are DNA/protein complexes located at the ends of chromosomes that are essential for chromosome stability. In normal somatic cells, telomeres decrease in length as the cells age (Harley et al., 1990, *Nature*, vol. 345(6274), pp. 458-60; Harley, C.B., 1991, *Mutat. Res.*, vol. 256(2-6), pp. 271-82; Levy et al., 1992, *J. Mol. Biol*., vol. 225(4), pp. 951-960; and Lindsey et al., 1991, *Mutat Res.*, vol. 256(1), pp. 45-8). This is due, in part at least, to the fact that DNA polymerases require an RNA primer and synthesize DNA only in the 5'-3' direction. Thus, the terminal RNA primer of the lagging strand represents information that is lost at each round of replication, as shown in Figure 1. Telomerase-mediated addition of telomere repeat sequences compensates for the loss of sequence information as a consequence of the inability of conventional DNA polymerase to fill in the gap left after removal of the terminal RNA primer. The natural process of telomere attrition has been suggested to play a role in programmed cell death (Harley et al.; Harley, C.B.; Levy et al.; Lindsey et al., cited above). In Henderson et al., 1989, *Mol. Cell. Biol*., vol. 9(1), pp. 345-348, the authors demonstrated that the telomeric G-strand overhang was present in phylogenetically diverse organisms. This supports the contention that this feature of telomeres is important for their normal function.

In Henderson et al., 1987, *Cell*, vol. 51(6), pp. 899-908, the authors showed that telomeric G-strand sequences form novel structures stabilized by G-G base pairs. This work gave rise to a number of related studies elucidating the G-quartet model for G-rich sequences (Cheong et al., 1992, *Biochemistry*, vol. 31(36), pp. 8406-8414; Kang et al., 1992, *Nature*, vol. 356, pp. 126-131; Sen et al., 1988, *Nature*, vol. 334, pp. 364-366; Sundquist, W.I., The structures of telomeric DNA, Nucleic Acids and Molecular Biology, 1991, Eckstein and Lilley ed., Springer-Verlag., Berlin; and Williamson et al., 1989, *Cell*, vol. 59(5), pp. 871-880). It has been suggested that these structures may play roles in fundamental cellular events including meiotic chromosome pairing, the control of gene expression and telomere-protein or telomere-telomere interactions.

In Ahmed et al., 1992, *Nuc. Acids Res*., vol. 20(3), pp. 507-511, the authors showed that the telomeric C-strand was capable of forming novel non-Watson-Crick structures at low and near neutral pH. These structures are either hairpin or duplex structures. It has been further suggested that the formation of such structures, even transiently, would facilitate the formation of stable G-4 DNA structures in the telomeric G-strand.

In Larson et al., 1987, *Cell*, vol. 50, pp. 477-483, the authors discovered that telomere length in *Tetrahymena* increases if cells are grown in continuous log phase. That is, in *Tetrahymena* spontaneous somatic mutations arise during prolonged log phase growth in which telomeres become short and less heterodisperse with respect to length. These mutants do not demonstrate the telomere length elongation capability seen with wild type cells and are, therefore, affected in some aspect of telomere length regulation.

In immortal cancer cells, however, telomeres remain constant in length over time, in contrast to the situation in normally aging cells (Takada et al., 1992, *Jpn. J. Cancer. Res.*, vol. 83(11), pp. 1124-1127; Counter et al., 1992, *Embo. J., vol*., 11(5), pp. 1921-1929; and Yu et al., 1990, *Nature,* vol. 344(6262), pp. 126-32). In fact, immortalized cancer cells are believed not to suffer the same telomere attrition observed in normal somatic cells or else they would suffer chromosome instability that, if not rectified, would lead to cell death. Avoiding telomere loss requires that telomere sequences be added to chromosomal termini. The enzyme responsible for telomere repeat addition, telomerase, has been identified only in immortal cell types (Takada et al., Counter et al., and Yu et al., cited above). The enzyme telomerase is directly responsible for the addition of telomeric DNA sequences to the ends of chromosomes. This enzyme contains both protein and RNA components. The RNA component encodes the telomeric DNA sequence. Hence, telomerase is a reverse transcriptase that adds telomere sequences to the ends of chromosomes using an internal RNA template.

Telomerase has been identified and partially purified from several human tumor cell lines but not from normal somatic cells. This has led to the hypothesis that telomerase is active in tumor cells, in which telomere length is constant and stable, and normally inactive in somatic cells whose telomeres shorten with time. Thus, the activity of telomerase constitutes a fundamental difference between tumor and normal cells and, as such, offers a tumor cell-specific target in the treatment of cancer by gene therapy.

In an immortal lower eukaryote, the ciliated protozoan *Tetrahymena thermophila*, the introduction of a mutant telomerase RNA (telRNA) gene resulted in the telomerase-mediated addition of mutant telomere sequences to the ends of chromosomes (Yu et al., 1990, *Nature*, vol. 344(62), pp. 126-32). This, in turn, rapidly led to genetic instability of the chromosomes and cell death. In these experiments the wild type telRNA was also present but was effectively competed by an approximately equal copy number of mutant telRNAs.

By altering the endogenous telomerase RNA sequence in tumor cells, one should be able to cause the addition of altered telomere sequences to chromosomes which would result in rapid cell death. However, this treatment should have no effect on normal somatic cells because telomerase is inactive in these cells. Therefore, the use of mutant human telRNAs could be an effective tumor cell-specific approach to cancer gene therapy.

### SUMMARY OF THE INVENTION

The primary object of the present invention is to provide for the identification and cloning of human telomerase RNA (telRNA), and to a method of using such telRNA as a tumor cell-specific target in the treatment of cancer by gene therapy.

The present invention provides for the identification and cloning of the RNA component of the telomerase enzyme, human telomerase RNA (telRNA). In another aspect of the invention, there is provided a method of using telRNA as a tumor cell-specific target in the treatment of various types of cancers by gene therapy.

In one embodiment of the invention, there is disclosed the identification of the human telomerase RNA (telRNA) gene. Using direct hybridization, an oligonucleotide probe is provided to screen cultured human tumor cells enriched in telRNA. Preferably, the probe is a 9-mer probe of the human telRNA gene sequence. Once a hybridizing size class is identified, a size-selected cDNA library is constructed and screened for the telRNA clone. The selected clone(s) will then be used to screen a human genomic library for isolation of the human telRNA gene.

In another embodiment of the present invention, there is disclosed the identification of the human telomerase RNA (telRNA) gene by PCR/hybrid selection. Here, additional telRNA sequence information is obtained from regions immediately flanking the telomere coding sequence using PCR/hybrid selection. A hybridization probe of 17-19 nucleotides is obtained in this manner. This probe is then used to screen a human genomic library for isolation of the telRNA gene.

Once the telRNA gene is isolated, it is mutated in vitro in a manner sufficient to compete with endogenous telRNA. That is, the mutant telRNA will encode for an aberrant telomeric DNA sequence. Preferably, the human telRNA gene of the invention is mutated by PCR-mediated mutagenesis.

The mutated telRNA gene has applications both *in vitro* and *in vivo*. For *in vitro* application, the mutated human telRNA gene of the invention can be cloned into a suitable expression vector, such as, for example, plasmid DNA, viral DNA including human viruses, animal viruses and insect viruses and bacteriophages to form a recombinant expression system which directs the expression of the mutated telRNA gene. Preferably, the expression vector is a mammalian expression vector. The expression vector, containing the mutated telRNA gene of the invention, is transfected (introduced) into human cancer cells, for example, HeLa cells and/or human melanoma cell lines, in culture, using conventional techniques known in the art. The transformed cells are thereafter collected and the effects of competition between the mutated telRNA gene and endogenous (natural) gene studied.

Alternatively, the mutated telRNA gene can be transfected i.e., delivered, into host (person) cancer cells, *in vivo*. The delivery system used to carry the mutated telRNA into the host can include any delivery system known in the art and/or to be developed, such as, for example, using liposome technology, a viral vector or producer cells. Once in the cells, it is expected that the mutant telRNA will compete, *in vivo*, with the endogenous telRNA gene in the formation of active telomerase, resulting in the addition of aberrant telomere sequences to the ends of chromosomes. This is predicted to cause genetic instability and rapid cell death. A key feature of the tumor cell-specific gene therapy approach of the present invention is that alteration of telomerase activity will have deleterious effects only on cells with active telomerase, such as tumor cells, but not cells in which telomerase is inactive, such as normal somatic cells.

### BRIEF DESCRIPTION OF THE DRAWINGS

Fig. 1 is a schematic diagram showing the role of telomerase in complete replication of chromosomal termini.

Fig. 2 is a schematic diagram showing the reiterative PCR procedure of the invention used to obtain additional telRNA sequence information (PrimBlock = duplexed region of the hybridization probe that will be used for PCR amplification of perfect hybrids; Degen = degenerate sites adjacent to telRNA coding sequence; TeloRepeat = the telRNA coding sequence complement).

Fig. 3 is a schematic diagram illustrating examples of the type of cloning vectors of the invention which are used for expression of mutated telRNA.

### DETAILED DESCRIPTION OF PREFERRED EMBODIMENTS OF THE INVENTION

As used throughout this specification, the following definitions apply for purposes of the present invention:

The term "gene" refers to those DNA sequences which transmit the information for and direct the synthesis of a single protein chain.

The term "telomerase RNA (telRNA) gene" refers to any gene which transmits the information for and directs the formation of active telomerase.

The term "transfecting" and/or "transfection" refers to any means for introducing i.e., delivering, the telRNA gene of the present invention into a cell. Examples of such means include infection, calcium phosphate precipitation and electroporation. It is understood that any delivery system known in the art is within the scope of the present invention. Examples of such delivery systems include a virus vector or plasmid vector. As used herein, the terms "transfecting" and "transforming" are interchangeable.

The term "infection" refers to the invasion by agents (e.g., viruses, bacteria, etc.) of cells where conditions are favorable for their replication and growth.

The present invention discloses the identification and cloning of the RNA component of the telomerase enzyme i.e., human telomerase RNA. The human telomerase RNA (telRNA) gene can be identified (isolated) by either direct hybridization using an oligonucleotide probe and/or by PCR/hybrid selection. Once the human telomerase RNA (telRNA) gene component is cloned, it is thereafter mutated *in vitro* so that it encodes an aberrant telomeric DNA sequence. The altered telRNA gene can be introduced into human tumor cells, in culture. The transformed cells i.e., those cells which express the mutant telRNA gene, can be collected, amplified and used to study the effects of competition with the natural telRNA gene.

Alternatively, the mutated telRNA gene can be transfected i.e., delivered, into host (person) cancer cells, *in vivo*. The delivery system used to carry the mutated telRNA into the host can include any delivery system known in the art and/or to be developed, such as, for example, using liposome technology, a viral vector or producer cells. Once in the cells, the mutant telRNA will compete with the endogenous telRNA in the formation of active telomerase, resulting in the addition of mutant telomere sequences to the ends of chromosomes. This will cause genetic instability and cell death. A key feature of the present invention, is the strategy that alteration of telomerase activity will have deleterious effects only on cells with active telomerase, such as tumor cells, but not cells in which telomerase is inactive, such as normal somatic cells.

In order to identify the human telRNA gene by direct hybridization, a 9-mer sequence 5'-TTGGGGTTG-3' (SEQ.ID.NO.1) which was used to identify the complement of the telomere sequence coding region of *Tetrahymena* telomerase was used as a starting point. Preliminary experiments with total RNA from *Tetrahymena* reveal that a band of the appropriate size (159 nucleotides) hybridizes to the *Tetrahymena* telRNA probe. This hybridization persists to 20°C but is lost at higher temperatures. Although preliminary, these results suggest that it is possible to identify the telRNA gene of *Tetrahymena* using the 9-mer probe (5'-TTGGGGTTG-3', SEQ.ID.NO.1), and that background hybridization is not significant. Based on known telRNA coding regions, the equivalent human sequence should be 5'-TTAGGGTTA-3' (SEQ.ID.NO.2). In *Tetrahymena*, 22 contiguous nucleotides are phylogenetically conserved flanking the telRNA coding sequence (Romero et al., 1991, Cell, vol. 67(2), pp. 343-53. Unfortunately, this conservation breaks down in more diverse species (Shippen-Lentz et al., "Functional evidence for an RNA template in telomerase", 1990, Science, vol. 247(4942), pp. 546-52). Therefore it is unlikely that the sequence flanking the telomere coding sequence in the human telRNA gene will resemble the known flanking sequence in *Tetrahymena*.

In this context, a PCR strategy is disclosed for providing the flanking sequence information for the human telRNA gene. Using PCR, a 9-mer telomere sequence with 9 flanking degenerate sites is hybridized to total RNA at high stringency. Only those molecules that remain hybridized under conditions requiring complete hybridization are recovered and amplified by PCR. After several rounds, the sequence of the amplified DNA is determined and used to isolate potential telRNA candidates from genomic and cDNA libraries from human cancer cells.

It is understood that any modifications i.e., insertions, deletions, mutations, recombinants, etc., of the gene encoding the telRNA of the invention are within the scope of the present invention provided that the modified sequence(s) encode for a gene, its homologs or a fragment thereof producing human telRNA.

The isolated telRNA gene may be mutated using PCR-mediated mutagenesis according to the method of Herlitze et al., 1990, *Gene*, vol. 91, pp. 143-147. Mutations may be introduced into the human telRNA gene at the telomere coding site prior to introduction of the gene into cultured human cancer cells. The altered sequence(s) are based on known or inferred telRNA coding sequence(s). In some cases the altered sequence(s) correspond to a telomere sequence from another organism while others are nontelomeric.

Recombinant DNA techniques are used to insert the mutant telRNA gene into an appropriate expression vector, i.e., a vector which contains the necessary elements for the transcription and translation of the inserted coding sequences. A large number of vector systems known in the art can be used, such as, plasmids, bacteriophage virus or other modified viruses. Suitable vectors include, but are not limited to the following viral vectors such as lambda vector system gt11, gtWES.tB, Charon 4, and plasmid vectors such as pBR322, pBR325, pACYC177, pACYC184, pAR series, pKK223-3, pUC8, pUC9, pUC18, pUC19, pLG339, pR290, pKC37, pKC101 and other similar systems. The mutant telRNA gene is cloned into the vector using standard cloning procedures in the art, as described by Maniatis et al., 1982, *Molecular Cloning: A Laboratory Manual*, Cold Springs Laboratory, Cold Springs Harbor, New York, and by Sambrook et al. 1989 edition of the same title.

The recombinant vector can then be introduced into the appropriate host cells, including but not limited to bacteria, virus, yeast, mammalian cells or the like. The vector system must be compatible with the host cell used. The recombinant vectors can be introduced into the host cells via transformation, transfection or infection using standard techniques in the art. A variety of host cell systems can be used to express the telRNA gene of the invention. For example, host cell systems include, but are not limited to the following: bacteria transformed with bacteriophage DNA, plasmid DNA or cosmid DNA such as *E. coli* JM103, *E. coli* C600, *E. coli* C04, *E. coli* DH20 and *E. coli* TB1; microorganisms such as yeast containing yeast vectors; mammalian cell systems infected with virus (vaccinia virus, adenovirus, retroviruses, etc.); insect cell systems infected with virus (baculovirus).

In order to obtain efficient expression of the telRNA gene, a promoter must be present. The promoter can include the natural telomerase promoter, a promoter in the expression system used or a combination thereof. RNA polymerase normally binds to the promotor and initiates transcription of a gene or a group of linked genes and regulatory elements (operon). Promoters vary in their strength, i.e., ability to promote transcription. For the purpose of expressing the gene of the invention, it is desirable to use strong promoters in order to obtain a high level of transcription and, hence, expression of the gene. Depending upon the host cell system utilized, any one of a number of suitable promoters can be used, such as, the lac promotor, trp promotor, recA promotor, ribosomal RNA promotor, the P_{R} and P_{L} promoters of coliphage lambda and others including but not limited to lacUV5, ompF, bla, lpp and the like, nos promoter, the small subunit ribulose bisphosphate carboxylase genes, the small subunit chlorophyll A/B binding polypeptide, the 35S promoter of cauliflower mosaic virus, and promoters isolated from plant genes, including the *Pto* promoter itself (Vallejos, et al., 1986, *Genetics*, vol. 112, pp. 93-105) to direct high levels of transcription of adjacent DNA segments. Additionally, a hybrid trp-lacUV5 (tac) promotor or other *E. coli* promoters produced by recombinant DNA or other synthetic DNA techniques can be used to provide for transcription of the gene of the invention.

When cloning in a eukaryotic host cell, enhancer sequences (e.g., the 72 bp tandem repeat of SV40 DNA or the retroviral long terminal repeats of LTRs, etc.) may be inserted to increase transcriptional efficiency. Enhancer sequences are a set of eucaryotic DNA elements that appear to increase transcriptional efficiency in a manner relatively independent of their position and orientation with respect to a nearby gene. Unlike the classic promotor elements (e.g., the polymerase binding site and the Goldberg-Hogness "TATA" box) which must be located immediately 5' to the gene, enhancer sequences have the remarkable ability to function upstream from, within, or downstream from eucaryotic genes. Therefore, the position of the enhancer sequence with respect to the inserted gene is less critical.

Any of the conventional cloning methods for insertion of the gene and/or gene fragment(s) into a vector can be used to ligate the promotor and other control elements into specific sites within the vector. Accordingly, gene sequences containing those regions coding for the telRNA of the invention can be ligated into an expression vector at a specific site in relation to the vector promotor and control elements so that when the recombinant DNA molecule is introduced into a host cell the foreign genetic sequence can be expressed (i.e., transcribed and translated) by the host cell.

As previously mentioned, the recombinant vector can be introduced into appropriate host cells (including but not limited to bacteria, virus, yeast, mammalian cells or the like) by transformation, infection or transfection (depending upon the vector/host cell system). Transformants are selected based upon the expression of one or more appropriate gene markers normally present in the vector, such as ampicillin resistance or tetracycline resistance in pBR322, or thymidine kinase activity in eucaryotic host systems. Expression of such marker genes should indicate that the recombinant DNA molecule is intact and is replicating. Expression vectors may be derived from cloning vectors, which usually contain a marker function. Such cloning vectors may include, but are not limited to the following: SV40 and adenovirus, vaccinia virus vectors, insect viruses such as baculoviruses, yeast vectors, bacteriophage vectors such as lambda gt-WES-lambda BC, lambda gt-1-lambda B, M13mp7, M13mp8, M13mp9, or plasmid DNA vectors such as pBR322, pAC105, pVA51, pACYC177, pKH47, pACYC184, pUB110, pMB9, pBR325, Col E1, pSC101, pBR313, pML21, RSF2124, pCR1, RP4, pBR328 and the like.

The expression vectors containing the telRNA gene can be identified by three approaches: (1) DNA-DNA hybridization using probes comprising sequences that are homologous to the gene(s); (2) presence or absence of "marker" gene function and (3) expression of inserted sequences based on physical, immunological or functional properties. Once a recombinant which expresses the gene is identified, the gene product should be analyzed. The ultimate goal is to use the gene or recombinant expression systems that express the gene in a method of gene therapy for treating various types of cancers. Once the telRNA gene is identified, it is cultured under conditions which facilitate growth of the cells and expression of the gene as will be apparent to one skilled in the art, then isolated and purified by standard methods including chromatography (e.g., ion exchange, affinity, and sizing column chromatography), centrifugation, differential solubility, or by any other standard techniques. In addition, the telRNA gene can be synthesized by chemical methods according to the procedure of Hunkapiller et al., 1984, *Nature*, vol. 310, pp. 105-111, once the sequence is identified.

The mutant telRNA is introduced into culture cells on plasmid vectors capable of integration into the genome and under inducible transcriptional control. In this way stable cell lines can be generated in which one could turn on the mutant telomerase gene and make mutant RNA *in vivo* in the cultured cells to test the effect of competition with the endogenous telomerase RNA.

For clinical application, the mutant telRNA gene can be carried i.e., delivered, into the host (person or animal), using any known transfection and/or delivery system in the art. For example, the delivery system can comprise a modified viral vector which is constructed in such a way as to allow it to be destroyed after successful cancer therapy, producer cells that make this vector and facilitate infection of surrounding cells or using liposome technology i.e., encapsulate the telRNA with a liposome. The present invention is further illustrated by the following examples.

### EXAMPLE I

**TELOMERASE ASSAY IN HeLa CELLS**

HeLa and other human tumor cell lines contain active telomerase (Morin, G.B., 1989, Cell, vol. 59(3), pp. 521-9; and Morin, 1991, Nature, vol. 353, pp. 454-456). To assay for telomerase activity crude extracts are prepared and assayed as described by Morin, G.B., 1989; and Morin, 1991, cited elsewhere herein. Briefly, an S-100 fraction is partially purified by DEAE ion exchange chromatography followed by gel filtration. Telomerase activity is assayed in a reaction containing the human G-strand telomeric primer 5'-TTAGGGTTAGGG-3' (SEQ.ID.NO.3), in the presence of dTTP, dATP, ³²P-dGTP and appropriate buffer components (Morin, G.B., 1989; and Morin, 1991, cited elsewhere herein). Telomerase-mediated primer elongation is evaluated by displaying products of the telomerase reaction by electrophoresis through a denaturing 8% polyacrylamide gel.

### EXAMPLE II

**IDENTIFICATION OF telRNA BY HYBRIDIZATION**

Nine nucleotides of the human telRNA gene sequence (i.e., the telomere repeat coding region which consists of about 1.5 repeat units, 5'-CAACAAA-3'(SEQ.ID.NO.4) in *Tetrahymena*, 5'-TAACCCTAA-3' (SEQ.ID.NO.5) in humans) were identified. An empirical approach can be taken to determine whether the 9-mer oligonucleotide probe can be used to identify the telRNA gene. Since the *Tetrahymena* telRNA gene has been cloned and telomerase from this organism can be highly purified (Greider, C.W., 1991, Mol. Cell Biol., vol. 11, pp. 4572-4580), the *Tetrahymena* system is used as a guide for evaluating the direct cloning and PCR strategies of the present invention.

The complement of the *Tetrahymena* telRNA sequence 5'-CAACCCCAA-3'(SEQ.ID.NO.6) i.e., 5'-TTGGGGTTG-3' (SEQ.ID.NO.7); 9-mer_{Tet}, can be synthesized and radiolabeled at the 5' end with ³²p using T4 polynucleotide kinase and γ³²P-ATP. Total cellular RNA and RNA isolated from partially purified telomerase can be prepared using a standard acid/phenol extraction method for telRNA purification from *Tetrahymena*. Purified RNA can be separated by electrophoresis on a 5% denaturing polyacrylamide gel and transferred electrophoretically to nylon membranes. The 9-mer probe can be hybridized to the immobilized RNA under low stringency conditions and the membranes washed at increasing temperatures with all other washing parameters being held constant. The 9-mer_{Tet} sequence has a predicted Tₘ of approximately 30°C in 6XSSC. The initial hybridization can be carried out at 7°C in 6XSSC and wash with 6XSSC at 15°, 20°, 25°, 30° and 35°C to empirically determine the approximate temperature at which the band corresponding to the *Tetrahymena* telRNA gene remains but other bands are lost. The number and size class of non-telRNA species that hybridize fully to the 9-mer_{Tet} probe can be evaluated.

In preliminary experiments, hybridization of the 9-mer_{Tet} probe to total cellular RNA and a control telRNA gene transcribed *in vitro* using a *Tetrahymena* telRNA plasmid (provided by Carol Greider, Cold Spring Harbor Laboratory, N.Y., N.Y.) has been observed. Two bands were seen in the total cellular RNA that corresponded to the telRNA band and a slightly larger species. These results suggest that it is possible to identify the desired species within a complex mixture of RNAs. Therefore, a similar procedure using the conditions previously described in the hybridization experiment in *Tetrahymena*, but with human RNA from HeLa cells can be carried out. Here, a 9-mer probe can be used that hybridizes to the human telRNA complement, 5'-TTAGGGTTA-3' (SEQ.ID.NO.2)(9-mer_{Hum}). Once a hybridizing size class has been identified, a size-selected cDNA library can be constructed and screened for telRNA clones as described hereinafter.

### EXAMPLE III

**IDENTIFICATION OF telRNA BY PCR**

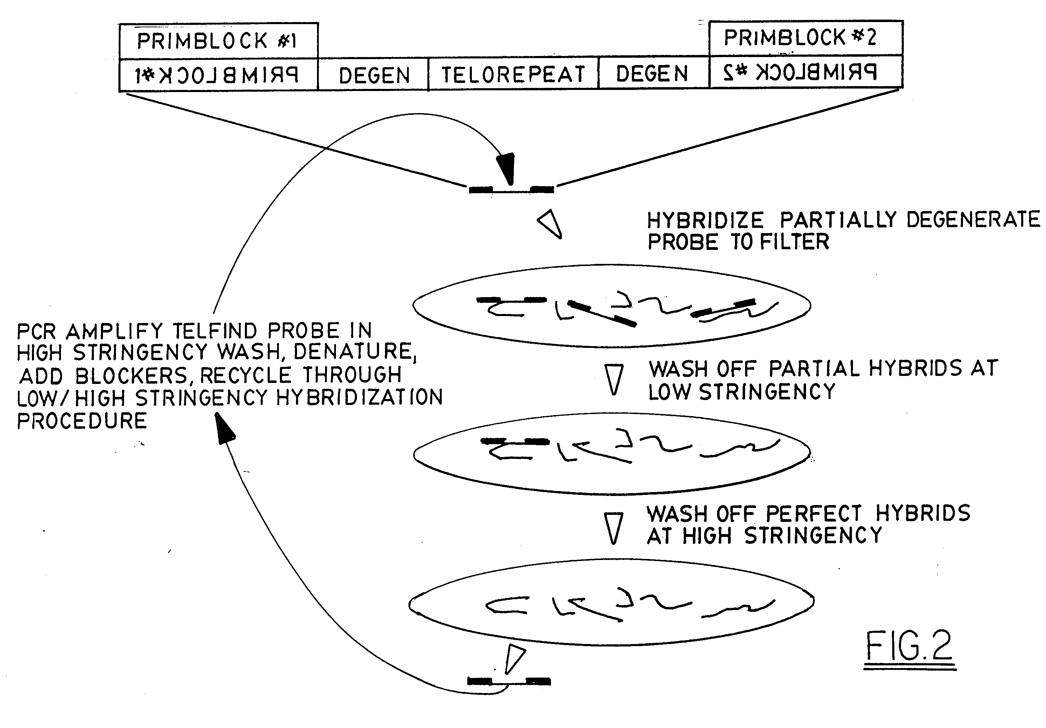

An alternative to isolating for the human telRNA gene using the 9-mer probe as set forth under Example II, is to obtain additional telRNA sequence information from regions immediately flanking the telomere coding sequence using a reiterative PCR/hybrid selection strategy. This strategy will provide a hybridization probe of about 17-19 nucleotides which should significantly facilitate screening of the human telRNA library. Here, immobilized RNA isolated from partially purified telomerase preparations can be used to select telRNA-specific oligonucleotides from a pool of partially degenerate synthetic DNA molecules. As shown in Figure 2, these synthetic molecules contain the complement of the telRNA coding region flanked on each side by 4-5 degenerate nucleotides and can serve as a substrate for cycles of hybrid selection and terminal PCR primer sequences that can be used to amplify the selected hybrids. The PCR primer region can be "blocked" by duplexing with complementary strands so that only the 9-mer_{Tet} region and the flanking degenerate sites are available for hybridization to the target RNA. RNA from human telomerase preparations can be immobilized on filter discs and initial hybridization with the pool of degenerate DNA oligonucleotides can be carried out under low stringency conditions. The filter discs can be washed at increasing stringency, and the oligonucleotides collected from the washed discs. Only molecules that were fully hybridized should be present in the high stringency washes. This material can be amplified by PCR and subjected to further rounds of hybrid-selection and amplification.

The end product will be a set of oligonucleotides, one of which is complementary to the human telRNA gene and whose sequence length is about 17-19 nucleotides. The sequence of these oligonucleotides can be determined following purification by cloning. As a control, this procedure can be carried out in parallel in *Tetrahymena.* This should determine whether the presence of degenerate flanking regions will contribute significantly to the number of non-telRNA sequences obtained. By isolating only fully hybridized molecules for PCR amplification, only those molecules that are fully duplexed with RNA on the filter discs and contain the telomere coding sequence should be amplified. In addition, since the degenerate regions comprise approximately half of the entire hybridizing sequence, the degenerate sequences by themselves should not hybridize at high stringency and therefore the presence of these sequences will not decrease the specificity of hybridization. The 17-19 nucleotide sequences obtained can be compared to the perfectly conserved flanking sequences in ciliates and the best candidates selected for genomic library screening. The cloned telRNA genes can be evaluated for their potential to encode for the telomere sequence as described hereinafter.

### EXAMPLE IV

**telRNA cDNA AND GENE CLONING**

The 9-mer probe can be used to screen a non-random telomere coding sequence-primed cDNA library. After sequence and secondary structure analysis of potential telRNA cDNAs, the best candidate(s) can be used to screen a genomic library. Isolation of the complete gene from a genomic library will be necessary since the cDNA strategy will produce only partial gene products. In addition, the 17-19 nucleotide probe generated by reiterative PCR in accordance with techniques described herein can be used to directly screen a human tumor cell genomic library for the telRNA gene.

Cellular RNA or RNA from partially purified telomerase preparations can be fractionated by electrophoresis and the size class(es) determined to hybridize to the telomere sequence probe can be used to generate a cDNA library according to the method of Ausubel et al., Current Protocols in Molecular Biology, 1&2, 1993. Since it would be premature to assume that the human telRNA is also a small Pol III transcript like the *Tetrahymena* telomerase RNA, the production of cDNAs will not be dependent upon particular characteristics of the RNA species other than its ability to hybridize with a telomere repeat probe.

RNA can be extracted by the acid/phenol procedure from whole cells or from partially purified telomerase preparations as described by Greider et al., 1985, *Cell*, vol. 43, pp. 405-413; Greider et al., 1987, *Cell*, vol. 51, pp. 887-898; Greider et al., "Characterization of the telomerase RNA from *Tetrahymena*", Symposium On The Molecular Biology Of RNA Held At The 17th Annual UCLA Symposia On Molecular And Cellular Biology, Keystone, Colorado, USA, April, 1988; and Greider et al., 1989, *Nature*, vol. 337(6205), pp. 331-337. The telomerase preparation from human cells is a 100,000 xg supernatant (S-100) extract which has been further purified by ammonium sulfate precipitation followed by DEAE ion exchange and gel filtration chromatography according to the method of Morin et al., 1989, Cell, vol. 59(3), pp. 521-529. RNAs of the size determined to hybridize to the telomere repeat sequence probe (9-mer_{Hum}) can be isolated from gel pieces and concentrated by precipitation with glycogen, if necessary (e.g. in the case of very low RNA concentrations).

First strand cDNA synthesis from the isolated RNA can be accomplished by the random hexamer priming procedure according to Ausubel et al., 1993, cited elsewhere herein. A variation of this method using the telRNA coding sequence can also be conducted as follows. The RNA strand of the resulting heteroduplex can be removed with RNAse H and second strand synthesis and blunt end formation can be carried out in the same reaction as described by Ausubel et al., 1993, cited elsewhere herein. Adapters can be ligated to the ends of the cDNAs and the cDNA purified by electrophoresis and cloned into the vector pSK+ (Stratagene). Plasmids can be introduced into a XLlBlue (Stratagene) strain of *E. coli* and plated. After colony hybridization, screening with the 9-mer probe at low stringency (about 20°C based on Northern experiments). The potentially positive colonies can be replated to obtain pure clones.

A variation on this procedure can be used to construct a cDNA library from the size-selected RNA using the 9-mer telRNA coding sequence to prime first strand synthesis. This is a redundant process since the 9-mer probe was the basis for selection of the RNA size class and should enrich at least 100-fold (Ausubel, 1993, cited elsewhere herein) for the cDNAs containing the telomere sequence. In this experiment, the highest stringency compatible with hybridization and enzyme requirements can be used during first strand synthesis to minimize the number of cDNAs resulting from partial duplex formation with the first strand synthesis primer.

The sequence of the inserts in the positive clones can be determined and their secondary structure (or at least that portion available from the region of the cDNA cloned) can be evaluated and compared to the conserved secondary structure for other telRNAs. telRNA secondary structure determination and phylogeny can be conducted according to the method of Romero et al., 1991, *Cell*, vol. 67(2), pp. 343-53.

A full-length telRNA clone is required for expression in human cells. Thus, the sequence information obtained from the telRNA clones of the invention can be used to isolate a genomic copy of the telRNA gene from either a complete or size-selected HeLa cell genomic library. The library can be purchased or constructed from restriction fragments of the size class shown to hybridize to the 17-19-mer probe by Southern analysis. The complete sequence of the putative telRNA gene can be determined and the secondary structure compared to that of other telRNAs as a test of whether the isolate is the human telRNA.

To test candidate telRNAs the following experiment can be carried out. RNase H cuts RNA only within a DNA/RNA hybrid. Sequence information from putative telRNAs can be used to generate DNA oligonucleotides that hybridize to a portion of the putative telRNA at a location(s) outside the telomere coding region. *In vitro* telomerase assays can be carried out in the presence or absence of the complementary DNA sequence and RNase H. If the assay shows that telomerase activity is significantly decreased in the presence of a particular oligonucleotide and RNase H, a positive identification of the telRNA gene can be recorded. This gene can then be used in subsequent experiments designed to alter telomerase activity in human tumor cells as described herein.

In an alternative embodiment, the 17-19 nucleotide sequence obtained by PCR can be used to directly screen a HeLa library, without the intermediate cDNA cloning step. Positive clones can be sequenced and the secondary structure of the encoded RNA evaluated as elsewhere herein. To determine the level of expression of the candidate telRNA in transformed cells, Northern analyses of human RNA can be conducted.

### EXAMPLE V

**ALTERNATIVE APPROACHES TO IDENTIFICATION OF THE HUMAN telRNA GENE**
**1. RNase Cleavage:**
   Telomerase RNA can be identified by cleavage of the RNA with RNase H when it is specifically hybridized to its DNA compliment. It is noted that some degree of biochemical purity is required prior to this step to permit identification and gel isolation of the cleaved RNA.
**2. Affinity Purification**:
   A human telomerase primer can be immobilized on an insoluble matrix material (e.g., paramagnetic beads) and an elongation reaction started. The beads are then isolated from the bulk cellular material. The attached telomerase with its telRNA component can be used to construct a cDNA library highly enriched for the telRNA gene. A cross-linking step can be added to physically but reversibly attach the telRNA to the telomerase primer to enhance its isolation.
**3. RNA Pol III Regulatory Region**:
   The coding sequence of telRNA and human RNA Pol III promoter or termination sequences can be used to PCR amplify the telRNA gene in accordance with the procedures described herein.
**4. Hybridization To Flanking Sequences:**
   Telomeres are added to specific sites at which chromosomal breaks occur (Harrington et al., 1991, Nature, vol. 353(3), pp. 451-454; and Morin, 1991, *Nature*, vol. 353, pp. 454-456). The sequence of this point is known for a form of α-thalassemia resulting from a chromosomal break and it is thus possible that this sequence hybridizes to a non-coding region of the telRNA adjacent to the coding region.

### EXAMPLE VI

**ENDOGENOUS telRNA EXPRESSION LEVELS**

The level of expression of the wild type telRNA in HeLa, A549 or other human tumor cells can be assessed by nuclease protection and Northern analyses. This will provide a measure of the required level of expression of mutated telRNAs necessary to compete with the endogenous telRNA. In previous studies in *Tetrahymena*, 10-20 fold over-expression of the telRNA gene (from a 50:1 ratio of gene copies) was sufficient to effectively compete with the endogenous gene (Yu et al., 1990, *Nature,* vol. 344(6262), pp. 126-32).

### EXAMPLE VII

**PCR-MEDIATED MUTAGENESIS**

Mutations in the telRNA gene can be introduced using PCR-mediated mutagenesis according to the method of (Herlitze et al., 1990, *Gene*, vol. 91, pp. 143-147). The coding sequence, 5'-TAACCCTAA-3' (SEQ.ID.NO.5) can be altered to generate both telomere sequences from other species and non-telomeric sequences as shown in Table II as follows:

**Table II**

| **TelRNA CODING SEQUENCE (5'-3')** | **SEQ.ID.NO.** | **SPECIES** |
|---|---|---|
| TAACCCTAA | SEQ.ID.NO.5 | Human (wild type) |
| TAATAA | SEQ.ID.NO.8 | Human G3 deletion |
| TTACCTTA | SEQ.ID.NO.9 | Human G1 deletion |
| TTACCTTA | SEQ.ID.NO.9 | Human C>G transition |
| TTACCCCTTA | SEQ.ID.NO.10 | Human C addition |
| CAACCCCAA | SEQ.ID.NO.11 | *Tetrahymena* (wild type) |
| CGACCCCAA | SEQ.ID.NO.12 | *Tetrahymena* mutation (*) |
| CAACCCCCAA | SEQ.ID.NO.13 | *Tetrahymena* mutation (*) |
| CAACCTCAA | SEQ.ID.NO.14 | *Tetrahymena* mutation (*) |
| CGACCCCAA | SEQ.ID.NO.12 | *Tetrahymena* mutation (*) |
| CAAAACCCCAAA | SEQ.ID.NO.15 | Euplotes |

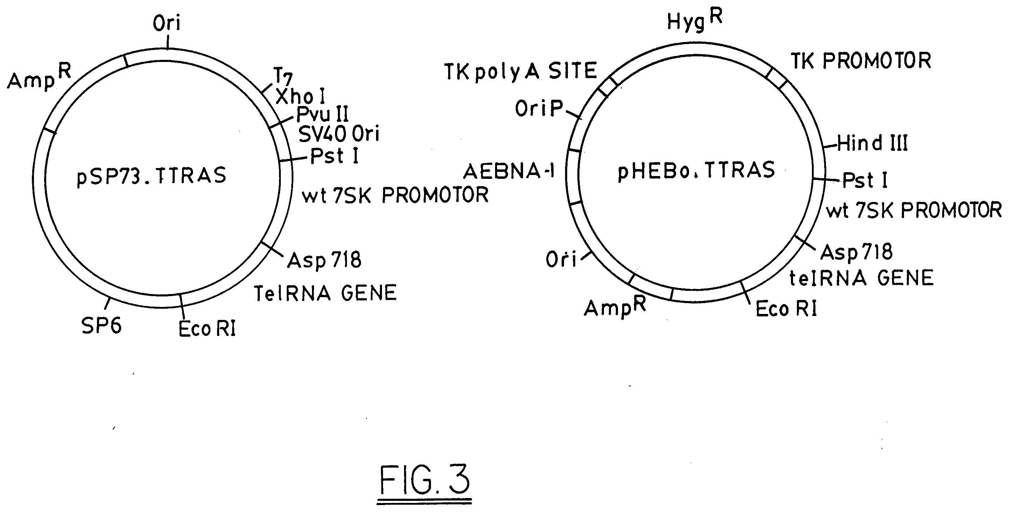

Mutations can be introduced into the human telRNA gene at the telomere coding site prior to introduction of the gene into cultured human cancer cells. The altered sequences are based on known or inferred telRNA coding sequences. In some cases the altered sequence corresponds to a telomere sequence from another organism while others are non-telomeric. *Tetrahymena* sequences with the superscript(*) have been shown to result in death of the cells containing the altered telRNA in just a few cell generations (Yu et al., 1990, cited elsewhere herein). The following protocol can be used: A mutagenic PCR primer (P1) containing perfect 5' and 3' matches and an internal mismatch in conjunction with a second (wild type sequence) primer (P2) is used to generate a portion of the desired product containing the altered sequence. This product is then purified and used in a second round of PCR with P2 and a third primer (P3) located at the opposite end of the gene to generate a full length mutant telRNA gene product. Products produced in this way can be cloned into an expression vector, for example, the mammalian expression vectors pSP73.TTRAS and pHEBo.TTRAS, as shown in Figure 3. The desired mutant telRNA clones can then be used to transfect the cultured human tumor cell lines.

### EXAMPLE VIII

**TRANSFECTION AND EXPRESSION**

Mammalian expression vectors were obtained that contain the *Tetrahymena* telRNA gene under the control of the strong 7SK Pol III promoter (pSP73.TTRAS and pHEBo.TTRAS; Fig. 3, Dr. Titia de Lange, Rockefeller University, N.Y., NY.). pHEBoTTRAS permits stable integration under hygromycin selection while pSP73.TTRS, lacking a drug resistance marker, can generate stable transfectants when co-transfected with a second plasmid carrying such a marker. Experiments in HeLa, A549 or other tumor cell lines can be conducted to determine whether the telRNA is efficiently transcribed. pSP73.TTRAS contains both T7 and SP6 promoters to permit transcription of sense and anti-sense RNAs *in vitro*.

The human telRNA gene can be cloned into the expression vectors at the same position as the *Tetrahymena* telRNA. Two convenient restriction sites, Asp718 and EcoRI are present in the flanking region of the *Tetrahymena* telRNA gene. Any manipulations of the human telRNA gene necessary to ensure that it will be correctly expressed by the 7SK promoter can be carried out using conventional cloning procedures in the art. The human telRNA gene construct in pHEBo.TTRAS can be introduced into HeLa cells using a modification of the CaPO₄ method according to Ausubel et al., 1993, cited elsewhere herein. Stable transfectants can be selected by growth in hygromycin. Levels of RNA expression can be assessed by Northern blotting or by nuclease protection assays (Ausubel et al., 1993, cited elsewhere herein) using a complimentary radiolabeled RNA transcript produced *in vitro* from pSP73.TTRAS. In both cases, telRNA can be quantitated by comparison with known concentrations of sense-strand telRNA prepared *in vitro*.

### EXAMPLE IX

**EFFECTS OF INTRODUCTION OF A MUTANT telRNA INTO CULTURED HUMAN CANCER CELLS**

Once transfected cells are obtained which produce the mutated telRNA, the effect of expression of a mutant telRNA gene on the viability of cultured human tumor cells can be determined. The growth rate and viability of the transfected cells known to contain mutant telRNA can be monitored. In previous studies, *Tetrahymena* cells harboring a mutant telRNA gene suffered growth and division defects that were phenotypically obvious. These included failure in cellular and nuclear division. Therefore, phenotypic alterations such as failure in nuclear division, and changes in cell shape and size in cells carrying the mutant telRNA gene(s) can be monitored. In the studies with *Tetrahymena*, it was observed that chromosomes acquired mutant telomere sequences encoded by the altered telRNA and their length regulation became unstable. Both increases and decreases in telomere length were observed (Yu et al., 1990, cited elsewhere herein). The effect on telomere length and sequence in transfected human tumor cells can be analyzed by Southern blotting, using the human wild type and mutant telomere sequences as probes. To determine whether the mutant telRNA gene is assembled into active telomerase complexes, telomerase can be partially purified from the transfected cells and assayed for its ability to generate wild type and mutant telomere sequences *in vitro*. Controls can be carried out in which the parent vector or a vector carrying the wild type telRNA gene can be used to transfect cells to distinguish any effects that are not the direct result of the presence of the mutant telRNA gene.

### EXAMPLE X

**CANCER GENE THERAPY**
**1. Mutant telRNA Method:**
   Once the human (or other) telomerase RNA has been cloned, the sequence in the RNA coding for the telomere can be modified so that when the wild type RNA is replaced with the mutant telomere sequences, foreign (mutant) sequence will be added to existing telomeres. This results in chromosome instability and rapid cell senescence and death. This effect would be specific for cancer cells since the telomerase enzyme is not active in normal cells.
   As previously described, for initial tests the mutant telomerase RNA can be introduced into culture cells on plasmid vectors capable of integration into the genome and under inducible transcriptional control. In this way stable cell lines can be generated in which the investigator can turn on the mutant telomerase gene and make mutant RNA *in vivo* at will to test the effect of competition with the endogenous telomerase RNA. For clinical application, the RNA gene can be carried into the host on a modified viral vector which is constructed in such a way as to allow it to be destroyed after successful cancer therapy, or in producer cells that make this vector and facilitate "infection" of surrounding cells in which the vector can not replicate.
**2. Ribozyme Method**:
   Ribozymes are RNA molecules with catalytic activity. One method to inactivate telomerase in cancer cells (telomerase is dormant in normal cells) is to destroy the essential RNA component of the enzyme complex. A ribozyme can be designed that will specifically cleave the telomerase RNA and thereby inhibit telomerase function. The ribozyme can be constructed based on known ribozyme structures of either the hammer head or *Tetrahymena* self splicing intron types. A guide sequence can be inserted into the function ribozyme that specifically binds to the telomerase RNA and positions it in the active site of the ribozyme.
   During the enzymatic cleavage of RNA by ribozymes, a portion of the cleaved RNA is covalently attached to the ribozyme. This can be used to isolate a portion of the sequence of the human telomerase RNA (currently not cloned) and the sequence information gained in this way used to isolate the complete gene. The only known sequence for the human telomerase RNA is inferred from the known telomere DNA sequence to include CCCTAA. This may be sufficient to generate a ribozyme that cleaves the RNA but is insufficient for use as a probe to isolate the gene by conventional methods.

Once a ribozyme with some functionality is demonstrated, modifications to the ribozyme can be carried out to "evolve" a very specific enzyme that cleaves only the telomerase RNA. This can be accomplished by methods recently developed that allow one to rapidly separate from a mixture of RNA sequences those which perform a desired task best, amplify them and further refine them. In clinical application, the ribozyme can be transcribed in vivo, and can be under inducible control. It is conceivable that a constitutive ribozyme of this type would be a preventative approach to cancer gene therapy since it can always be present to inactivate telomerase RNA should a normal cell undergo transformation.

Although the invention has been described in detail for the purpose of illustration, it is understood that such detail is solely for that purpose, and variations can be made therein by those skilled in the art without departing from the spirit and scope of the invention which is defined by the following claims.

## Claims

1. An isolated gene or gene fragment comprising a DNA molecule which encodes for human telomerase RNA.

2. The gene according to claim 1, wherein said DNA molecule comprises cDNA and genomic RNA.

3. A recombinant DNA vector comprising a DNA sequence or a mutant DNA sequence thereof, encoding human telomerase RNA.

4. The vector according to claim 3, wherein said vector is selected from the group consisting of a plasmid vector, virus vector and bacteriophage.

5. The vector according to claim 4, wherein said vector is a mammalian vector.

6. The vector according to claim 5, wherein said DNA sequence is inserted into said vector in proper orientation.

7. The vector according to claim 6, wherein said DNA sequence is operatively linked to a promotor sequence.

8. The vector according to claim 7, wherein said DNA sequence comprises the DNA molecule according to claim 1.

9. A cell transformed with the recombinant DNA vector according to Claim 3 or 8.

10. The cell according to claim 9, wherein said cell is a human tumor cell.

11. A method of expressing human telomerase RNA in a host cell, comprising:
transforming a host cell with the isolated gene or gene fragment according to claim 1 under conditions which facilitate expression of the gene or gene fragment in said host cell, thereby producing human telomerase RNA.

12. Mutated human telomerase RNA, or DNA encoding it, for use in medicine.

13. The use of mutated human telomerase RNA, or DNA encoding it, in the preparation of a medicament for the treatment of diseases involving tumors.
